# EUROPÄISCHE PATENTSCHRIFT

(11) **EP 3 570 721 B1**
(45) Veröffentlichungstag und Bekanntmachung des Hinweises auf die Patenterteilung: **30.06.2021**
(21) Anmeldenummer: 18703619.9
(22) Anmeldetag: 17.01.2018
(51) Int. Cl.: A61B 1/00

(54) **ENDOSKOP MIT EINEM ENDOSKOPKOPF UND EINEM ENDOSKOPKOPF ANBRINGBAREN ALBARRANHEBEL**
ENDOSCOPE HAVING AN ENDOSCOPE HEAD AND AN ALBARRAN LEVER, WHICH IS ATTACHABLE TO THE ENDOSCOPE HEAD
ENDOSCOPE MUNI D'UNE TÊTE D'ENDOSCOPE ET D'UN LEVIER D'ALBARRAN POUVANT ÊTRE MONTÉ SUR LA TÊTE D'ENDOSCOPE

(30) Priorität: 18.01.2017 DE 102017100868
(43) Veröffentlichungstag der Anmeldung: 27.11.2019
(73) Patentinhaber: Hoya Corporation, Tokyo 160-8347 (JP)
(72) Erfinder: DO, Anh, Minh, 80689 München (DE)
(74) Vertreter: TBK
(86) Internationale Anmeldenummer: PCT/IB2018/000019
(87) Internationale Veröffentlichungsnummer: WO 2018/134670

(56) Entgegenhaltungen:
- DE-A1-102016 105 033
- JP-A- H08 243 076
- US-A- 5 707 344

## Beschreibung

Die vorliegende Erfindung bezieht sich auf ein Endoskop mit einem Endoskopkopf und einem am Endoskopkopf anbringbaren Albarranhebel.

Ein solcher Albarranhebel kann bei einem Endoskop zur Untersuchung z.B. der Speiseröhre oder auch des Duodenums, des Gallengangs, der Galle, des Bauchspeicheldrüsengangs, der Bauchspeicheldrüse etc., angewendet werden.

Ein solches Endoskop hat eine Optik (Beleuchtungseinrichtung und Kamera). Das Endoskop hat außerdem am Ausgang des Arbeitskanals den Albarranhebel, der durch Verschwenken eine gezielte Umlenkung der Werkzeuge ermöglicht, die durch den Arbeitskanal geschoben werden.

Nach der Anwendung des Endoskops wird dieses einer Aufbereitung unterzogen. Diese muss zuverlässig die Übertragung sämtlicher Keime oder Mikroorganismen wie Bakterien, Viren, Pilze, Würmer aber auch Sporen ausschließen. Bei der Aufbereitung wird zunächst das Endoskop manuell gereinigt, um organisches Material oder chemische Rückstände rückstandsfrei zu entfernen. Nach der Reinigung erfolgt eine maschinelle Desinfektion oder Sterilisation. Somit soll vermieden werden, dass Keime oder Mikroorganismen etc., mit denen ein Endoskop bei einem Einsatz des Endoskops in Kontakt gelangte, beim nächsten Einsatz auf den Patienten übertragen werden.

Beispielsweise offenbart die DE 196 27 016 C1 ein Endoskop mit einem Albarranhebel. Genauer gesagt hat das Endoskop einen vom Endoskop lösbaren Träger, in dem der Albarranhebel schwenkbar an einer im Träger abgestützten Achse angeordnet ist. Das Schwenken des Albarranhebels wird über ein Zugseil bewerkstelligt, das am Albarranhebel verankert ist und im Endoskop geführt wird. Ferner offenbart die DE 10 2016 105 033 A1 ein Endoskop mit einem Endoskopkopf und einem am Endoskopkopf anbringbaren Albarranhebel gemäß dem Oberbegriff von Anspruch 1.

Die vorliegende Erfindung hat die Aufgabe, ein Endoskop zu schaffen, bei dem noch besser vermieden wird, dass Keime, mit denen ein Endoskop in Kontakt gelangte, beim nächsten Einsatz auf den Patienten übertragen werden.

Diese Aufgabe ist durch ein Endoskop mit den Merkmalen von Anspruch 1 gelöst. Ein alternatives Endoskop ist in Anspruch 2 aufgezeigt. Vorteilhafte Weiterbildungen sind Gegenstand der abhängigen Ansprüche.

Im erfindungsgemäßen Endoskop weist ein an einem Endoskopkopf anbringbarer Albarranhebel eine Werkzeugleitfläche, an der ein durch einen Arbeitskanal eines Endoskops führbares Werkzeug in Kontakt treten kann, und eine Rastvorrichtung zum formschlüssigen Einrasten an einem von einer proximalen Seite des Endoskops betätigbaren Kraftübertragungsabschnitt des Endoskopkopfes des Endoskops auf, wobei der Kraftübertragungsabschnitt eine Kraft zum Schwenken des Albarranhebels auf den Albarranhebel überträgt.

Ein solcher Albarranhebel kann mit Leichtigkeit am Endoskopkopf eingerastet und wieder gelöst werden. Somit kann der Albarranhebel als Einmalverwendungsobjekt konzipiert werden. Ein solcher Albarranhebel kann nach einmaliger Anwendung entsorgt werden und durch einen frischen Albarranhebel ersetzt werden. Dadurch kann sichergestellt werden, dass der Albarranhebel keine Keime übertragen kann, wenn das Endoskop beim nächsten Patienten eingesetzt wird. Der benutzte Albarranhebel kann natürlich auch einer Reinigung und Sterilisation zugeführt werden.

Die Rastvorrichtung kann an der proximalen Seite des Albarranhebels eine geometrische Form aufweisen, die einer Gegenform eines Wellenabschnittes des Kraftübertragungsabschnittes des Endoskopkopfes entspricht. Auf diesen Wellenabschnitt ist der Albarranhebel aufsetzbar. Ein Rastmittel der Rastvorrichtung kann ein Entfernen des Albarranhebels vom Wellenabschnitt des Kraftübertragungsabschnittes des Endoskopkopfes verhindern. Somit kann der Albarranhebel mit einem sehr einfachen Aufbau am Endoskopkopf eingerastet und wieder gelöst werden. Das formbezogene Einrasten verleiht dem eingerasteten Zustand eine ausreichende Sicherheit vor einem versehentlichen Entfernen des Albarranhebels vom Endoskopkopf.

Das Rastmittel kann an der zur Werkzeugleitfläche entgegengesetzten Seite des Albarranhebels vorgesehen sein. Somit beeinträchtigen die Elemente, die beim Einrasten des Albarranhebels involviert sind, nicht ein zwischen einem Arbeitskanal und der Werkzeugleitfläche geführtes Werkzeug.

Gemäß Anspruch 1 ist das Rastmittel als am Albarranhebel vorgesehener zum Albarranhebel relativ bewegbarer Einrasthebel ausgebildet. Der Einrasthebel kann zum Albarranhebel drehbar sein. Der Albarranhebel kann an der der zur Werkzeugleitfläche entgegengesetzten Seite des Albarranhebels eine Drehwelle haben, wobei der Einrasthebel an der Drehwelle relativ zum Albarranhebel drehbar ist.

Alternativ ist gemäß Anspruch 2 das Rastmittel als am Albarranhebel vorgesehener zum Albarranhebel verschiebbarer Schieber ausgebildet. Der Schieber kann zum Albarranhebel verschiebbar sein.

Das Rastmittel kann an der der zur Werkzeugleitfläche entgegengesetzten Seite des Albarranhebels eine Betätigungseinrichtung zur Betätigung des Rastmittels haben. Somit ist auch das Rastmittel so am Albarranhebel angeordnet, dass es keine Beeinträchtigung für ein zwischen einem Arbeitskanal und der Werkzeugleitfläche geführtes Werkzeug darstellt. Eine solche Betätigungseinrichtung ist bei einem Einrasthebel oder einem Schieber anwendbar.

Der Albarranhebel kann aus Kunststoff hergestellt sein. Somit ist er kostengünstig. Daher ist er als Einmalverwendungsbauteil gut geeignet.

Am erfindungsgemäßen Endoskopkopf können ein Wellenabschnitt des Kraftübertragungsabschnittes des Endoskopkopfes und die Rastvorrichtung des Albarranhebels eine formschlüssige Verbindung eingehen, durch die die Kraft zum Schwenken des Albarranhebels vom Endoskopkopf zum Albarranhebel übertragbar ist.

Die geometrische Form der Rastvorrichtung des Albarranhebels kann eine Gegenform zur geometrischen Form des Wellenabschnittes des Kraftübertragungsabschnittes des Endoskopkopfes bilden.

Das Endoskop kann eine am Aussenumfang des Endoskopkopfes von der distalen Seite aufsetzbare Kappe haben. Somit wird der distale Abschnitt des Endoskopkopfes und der Albarranhebel durch die Kappe geschützt. Die ist Kappe von einem Zugseil zur Betätigung des Albarranhebels separat. In der von der distalen Seite auf den Endoskopkopf aufgeschobenen Kappe ist dann der Albaranhebel relativ zur Kappe schwenkbar angeordnet. Die Kappe hat eine Öffnung, durch die Mikrowerkzeuge geschoben werden können. Die Kappe ist an der distalen Seite des Endoskopkopfes lösbar montierbar.

Das Endoskop kann ein Duodenoskop sein.

Die vorstehend erläuterten Aspekte der vorliegenden Erfindung können geeignet kombiniert werden.

### Kurzbeschreibung der Zeichnungen

Fig. 1 zeigt eine schematische perspektivische Ansicht eines Endoskopkopfes mit einem Albarranhebel eines Ausführungsbeispiels der vorliegenden Erfindung.
Fig. 2 zeigt eine schematische perspektivische Ansicht des Endoskopkopfes von Fig. 1, wobei der Albarranhebel gelöst ist.
Fig. 3 zeigt eine schematische perspektivische Ansicht des Endoskopkopfes mit dem Albarranhebel von Fig. 1, wobei aus Gründen der Übersichtlichkeit das Gehäuse des Endoskopkopfes weggelassen wurde.
Fig. 4 zeigt eine schematische perspektivische Ansicht ähnlich wie in Fig. 3, wobei der Albarranhebel gelöst ist.
Fig. 5 zeigt eine schematische Seitenansicht des Endoskopkopfes mit eingebautem Albarranhebel.
Fig. 6 zeigt eine schematische Seitenansicht des Endoskopkopfes mit nicht eingebautem Albarranhebel.
Fig. 7 zeigt eine schematische Seitenansicht des Albarranhebels des Ausführungsbeispiels in einem Zustand, bei dem der Einrasthebel eingerastet ist.
Fig. 8 zeigt eine schematische Seitenansicht des Albarranhebels des Ausführungsbeispiels in einem Zustand, bei dem ein Einrasthebel nicht eingerastet ist.
Fig. 9 zeigt eine schematische perspektivische Ansicht des Albarranhebels des Ausführungsbeispiels in einem Zustand, bei dem der Einrasthebel eingerastet ist.
Fig. 10 zeigt eine schematische perspektivische Ansicht des Albarranhebels des Ausführungsbeispiels in einem Zustand, bei dem ein Einrasthebel nicht eingerastet ist.

Nachstehend ist die vorliegende Erfindung detailliert unter Bezugnahme auf die Zeichnungen anhand von Ausführungsbeispielen beschrieben.

### Ausführungsbeispiel 1

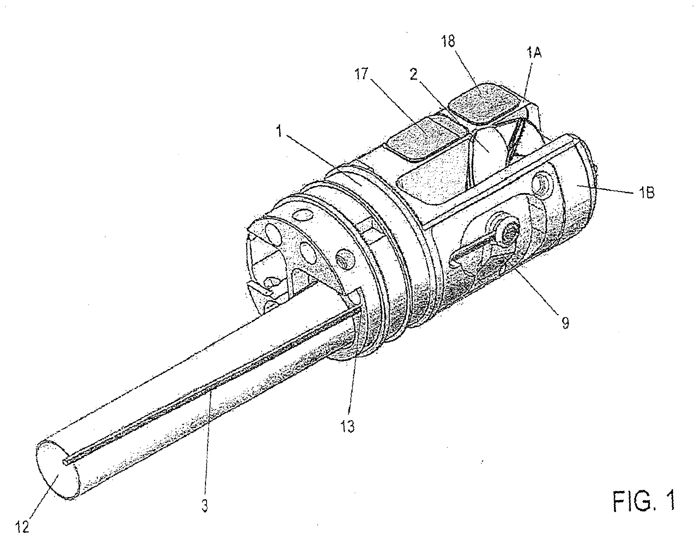

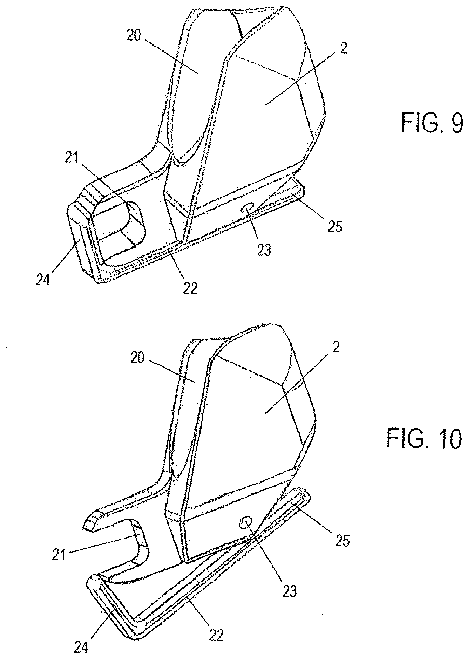

Nachstehend ist unter Bezugnahme auf die Figuren 1 bis 10 ein erstes Ausführungsbeispiel der vorliegenden Erfindung beschrieben.

Zunächst ist unter Bezugnahme auf die Figuren 1 bis 6 ein erfindungsgemäßer Endoskopkopf 1 beschrieben.

Der erfindungsgemäße Endoskopkopf 1 ist als zylindrischer Körper aufgebaut und weist einen Arbeitskanal 12 und einen Seilzugkanal 13 auf, die sich jeweils entlang der Längsrichtung des Endoskopkopfes 1 und parallel zueinander erstrecken. Der Seilzugkanal 13 führt ein Zugseil 3 zur Betätigung eines Albarranhebels 2. Der Arbeitskanal 12 führt Mikrowerkzeuge zur Untersuchung z.B. der Speiseröhre oder auch des Duodenums, des Gallengangs, der Galle, des Bauchspeicheldrüsengangs, der Bauchspeicheldrüse etc.

An der distalen Seite hat der Endoskopkopf 1 eine Optikerstreckung 1A, an der eine Kamera 17 und eine Beleuchtungseinrichtung 18 in bekannter Weise vorgesehen sind, wobei diese Optikerstreckung in Fig. 1 an der hinteren Seite der perspektivischen Ansicht gezeigt ist.

Der Arbeitskanal 12 endet in einem Abschnitt des Endoskopkopfes 1, der vom distalen Ende beabstandet ist, und bildet dort eine distale Ausgangsöffnung des Arbeitskanals.

Distal vom distalen Ausgang des Arbeitskanals 12 wird der Albarranhebel 2 angeordnet, der relativ zum Endoskopkopf 1 schwenken kann. Der Arbeitskanal 12 verläuft somit in distaler Richtung zum Albarranhebel 2 hin.

An der distalen Seite hat der Endoskopkopf 1 eine Albarranhebelabstützerstreckung 1B, die in Fig. 1 an der vorderen Seite der perspektivischen Ansicht gezeigt ist. An der Albarranhebelabstützerstreckung 1B ist eine Schwenkwelle 10 so gelagert, dass sie sich zu einem Innenraum zwischen der Optikerstreckung 1A und der Albarranhebelabstützerstreckung 1B vorragt. Die Schwenkwelle 10 bildet einen Kraftübertragungsabschnitt, der eine Drehbewegungskraft auf den Albarranhebel 2 aufbringt. Die Schwenkwelle 10 ist quasi die Drehwelle des Albarranhebels 2.

Genauer gesagt wird die Drehbewegungskraft für den Albarranhebel 2 durch das Zugseil 3 aufgebracht, dessen Seilzugnippel an einem Ende eines Hebelelementes 9 gekoppelt ist. Das andere Ende des Hebelelementes 9 ist mit dem in der Albarranhebelabstützerstreckung 1B gelagerten Ende der Schwenkwelle 10 einstückig verbunden. Das entgegengesetzte Ende der Schwenkwelle 10 bildet einen aus der Albarranhebelabstützerstreckung 1B zur Optikerstreckung 1A vorragenden Wellenabschnitt 11 aus.

Der Albarranhebel 2 wird auf diesen in den Innenraum zwischen der Optikerstreckung 1A und der Albarranhebelabstützerstreckung 1B hineinragenden Wellenabschnitt 11 der Schwenkwelle 10 aufgesetzt. Der Wellenabschnitt 11 ist so positioniert, dass er der distalen Ausgangsöffnung des Arbeitskanals 12 gegenüberliegt.

Die Schwenkwelle 10 hat somit ein Ende, das im Inneren der Albarranhebelabstützerstreckung 1B angeordnet ist. An dem zum Wellenabschnitt 11 entgegengesetzten Ende der Schwenkwelle 10 greift über das Hebelelement 9 das Zugseil 3 an. Anders ausgedrückt ist das distale Endes des Zugseils 3 mit dem zum Wellenabschnitt 11 entgegengesetzten Ende der Schwenkwelle 10 wirkverbunden. Ein in proximaler Richtung erfolgendes Ziehen des Zugseils 3 bewirkt somit ein Drehen der Schwenkwelle 10.

Das zum Wellenabschnitt 11 entgegengesetzte Ende der Schwenkwelle 10 ist gegenüber der Umgebung abgedichtet. Das Zugseil 3 ist im Seilzugkanal angeordnet. Der Seilzugkanal ist ebenfalls gegenüber der Umgebung abgedichtet. Somit hat das Zugseil 3 keinen Kontakt mit der Umgebung des Endoskopkopfes 1. Ein abgedichteter Raum, in dem das distale Ende des Zugseils 3, das Hebelelement 9 und das zum Wellenabschnitt 11 entgegengesetzte Ende der Schwenkwelle 10 angeordnet sind, ist in der Albarranhebelabstützerstreckung 1B vorgesehen. Dieser abgedichtete Raum ist lediglich zur proximalen Seite hin über den Seilzugkanal offen.

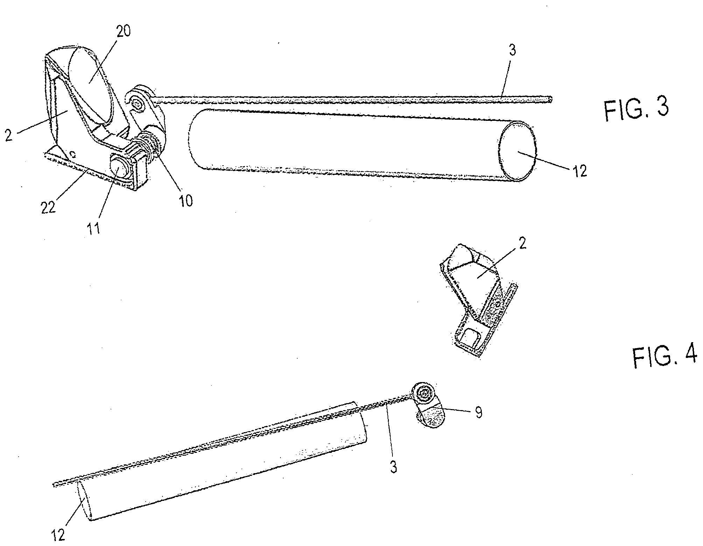

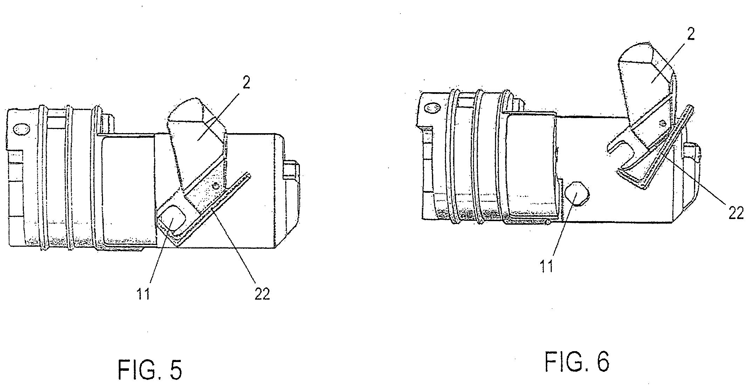

Der Wellenabschnitt 11 der Schwenkwelle 10 hat eine Form eines geraden Prismas mit einem Vieleck als Grundfläche in Längsrichtung der Schwenkwelle 10. Z.B. kann als Form des Wellenabschnittes 11 ein Dreikant oder Vierkant gewählt werden. Im vorliegenden Ausführungsbeispiel ist der Wellenabschnitt 11 in Vierkantform ausgebildet, siehe Figuren 3 und 6.

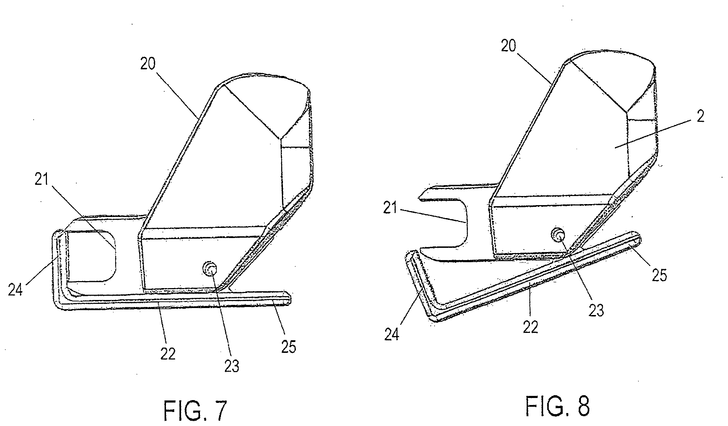

Nachstehend ist der Albarranhebel 2 unter Bezugnahme auf die Figuren 7 bis 10 beschrieben.

An der proximalen Seite weist der Albarranhebel 2 eine Einpassform 21 auf, die eine Gegenform zu der Form des Wellenabschnittes 11 bildet. Die Einpassform 21 muß dazu in der Lage sein, eine Drehung der Schwenkwelle 10 auf den Albarranhebel 2 zu übertragen. Im vorliegenden Ausführungsbeispiel ist die Einpassform 21 in Vierkantform ausgebildet, wie dies in Fig. 5 und in den Figuren 7 bis 10 gezeigt ist.

Der Albarranhebel 2 wird so am Endoskopkopf 1 eingebaut, dass die Einpassform 21 in die proximale Richtung weist. Die Einpassform 21 umgibt bei auf dem Wellenabschnitt 11 aufgeschobenem Albarranhebel 2 die Umfangsfläche des Wellenabschnittes 11 von drei Seiten, d.h. in etwa von oben, von der distalen Seite und von unten.

Der Albarranhebel 2 hat eine Werkzeugleitfläche 20, an der ein durch den Arbeitskanal des Endoskops führbares Werkzeug in Kontakt treten kann. Im Ausführungsbeispiel verläuft die Werkzeugleitfläche 20 am Albarranhebel 2 schräg, d.h. sie ist zur proximalen Seite und zur nach oben weisenden Seite des Albarranhebels 2 in den Figuren 7 bis 10 gewandt.

An der Einpassform 21 greift eine Rastnase oder Sperrnase 24 eines Einrasthebels 22 an.

Der Einrasthebel 22 bildet ein Rastmittel der vorliegenden Erfindung und bildet zusammen mit der Einpassform 21 eine Rastvorrichtung der vorliegenden Erfindung.

Der Einrasthebel 22 ist über eine Drehwelle 23 mit dem Albarranhebel 2 verbunden. Der Einrasthebel 22 kann mittels der Drehwelle 23 relativ zum Albarranhebel 2 geschwenkt werden.

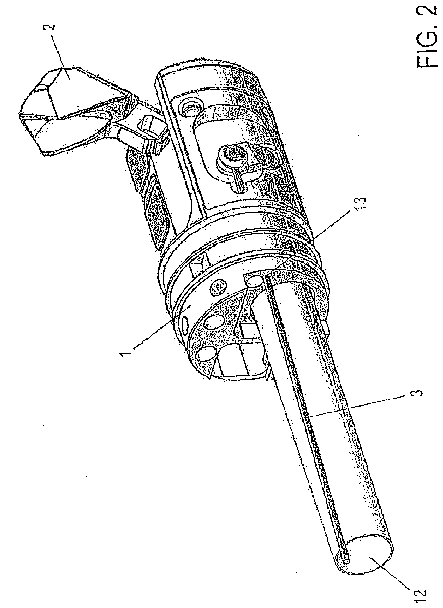

Sowohl der Einrasthebel 22 als auch die Drehwelle 23 sind an der zur Werkzeugleitfläche 20 entgegengesetzten Seite des Albarranhebels 2 (d.h. an der unteren Seite des Albarranhebels 2 in Fig. 2) vorgesehen. Das distale Ende des Einrasthebels 22 bildet eine Betätigungseinrichtung 25. Wenn das distale Ende des Einrasthebels 22 zum Albarranhebel 2 hin gedrückt wird, schwenkt der Einrasthebel 22 so, dass die Rastnase 24 sich von der Einpassform 21 wegbewegt. Wenn das distale Ende des Einrasthebels 22 vom Albarranhebel 2 weg bewegt wird, schwenkt der Einrasthebel 22 so, dass die Rastnase 24 sich zu der Einpassform 21 hin bewegt. Im letzteren Fall wird die proximale Seite der Einpassform 21 durch die Rastnase 24 geschlossen, siehe Fig. 7. Vorzugsweise ist in dem Bereich distal der Drehwelle 23 eine nicht gezeigte Drängvorrichtung wie z.B. eine Feder (Blattfeder oder Spiralfeder) angeordnet, die den distalen Abschnitt des Einrasthebels 22 vom Albarranhebel 2 weg vorspannt. Anders ausgedrückt, wird auf den Einrasthebel 22 eine Vorspannung aufgebracht, die die Rastnase 24 schließt. Somit ist bei nicht betätigtem Einrasthebel 22 die Einpassform 21 durch die Rastnase 24 geschlossen. D.h. Fig. 7 zeigt den nicht betätigten Zustand.

Bei auf dem Wellenabschnitt 11 aufgeschobenem Albarranhebel 2 liegt somit eine distale Fläche der Rastnase 24 an einer proximalen Fläche der Umfangsfläche des Wellenabschnittes 11 an.

Wenn die Rastnase 24 des Einrasthebels 22 an der Einpassform 21 in der auf den Wellenabschnitt 11 aufgeschobenen Stellung schließt oder arretiert, ist die Umfangsfläche des Wellenabschnittes 11 von der Rastnase 24 und der Einpassform 21 umgeben. Die distale Fläche der Rastnase 24 verhindert, dass der Albarranhebel 2 in distaler Richtung vom Endoskopkopf 1 abgezogen werden kann.

Die Bestandteile des Albarranhebel 2 sind aus Kunststoff hergestellt. Der Albarranhebel 2 kann aus Kunststoff beispielsweise mittels 3D-Drucker oder Spritzformen hergestellt. Durch die Herstellung mittels 3D-Drucker oder Spritzformen kann der Albaranhebel genau aber noch unter geringen Kosten hergestellt werden. Weitere geeignete Herstellverfahren können aufgegriffen werden, solange diese eine genaue und kostengünstige Produktion erlauben.

### Funktion der Erfindung

Beim Aufsetzen des erfindungsgemäßen Albarranhebel 2 an den Wellenabschnitt 11 der Schwenkwelle 10 des Endoskopkopfes 1 wird der Albarranhebel 2 so in den Innenraum zwischen der Optikerstreckung 1A und der Albarranhebelabstützerstreckung 1B eingeführt, dass die Einpassform 21 des Albarranhebels 2 in die proximale Richtung zeigt. Der Einrasthebel 22 wird durch Drücken des Betätigungsabschnittes 25 geöffnet, siehe Fig. 6 und 8, womit die Einpassform 21 des Albarranhebels 2 auf den Wellenabschnitt 11 der Schwenkwelle 10 geschoben werden kann. Daraufhin wird der Einrasthebel 22 geschlossen, wobei die Rastnase 24 an der proximalen Seite des Wellenabschnittes 11 (in Fig. 5 links unterhalb des Wellenabschnittes 11) vor den Wellenabschnitt 11 geschoben wird. Somit ist der Albarranhebel 2 an dem Wellenabschnitt 11 der Schwenkwelle 10 eingerastet.

Wenn der Albarranhebel 2 wie vorstehend beschrieben am Endoskopkopf 1 angeordnet ist, kann eine nicht gezeigte Kappe auf den distalen Aussenumfang des Endoskopkopfes 1 gesetzt werden. Diese Kappe erleichtert das Einführen des Endoskopkopfes 1 beim Patienten. Die Kappe weist eine Öffnung an der nach oben weisenden Seite von Fig. 1 auf, d.h. an der Seite, zu der durch den Albarranhebel 2 die Mikrowerkzeuge geschoben werden und in die die Kamera 17 und Beleuchtungseinrichtung 18 weisen.

Nach dem Einsatz des Endoskops werden der Albarranhebel 2 und die Kappe entfernt. Dabei wird durch Drücken des Betätigungsabschnittes 25 die Rastnase 24 von der proximalen Seite des Wellenabschnittes 11 weggeschoben. Somit kann der Albarranhebel 2 vom Wellenabschnitt 11 der Schwenkwelle 10 in die distale Richtung abgezogen werden. Der Albarranhebel 2 und die Kappe können entsorgt werden. Lediglich das Endoskop mit dem Endoskopkopf 1 werden der Reinigung und Sterilisation übergeben.

### Wirkungen der Erfindung

Der Albarranhebel 2 kann als separate steril verpackte Baugruppe vorgesehen werden, auf die ein Bediener des Endoskops zugreift.

Der Albarranhebel 2 ist leicht am Endoskopkopf 1 montierbar und demontierbar. Die Rastvorrichtung aus dem Einrasthebel 22 und der Einpassform 21 geht mit dem Wellenabschnitt 11 der Schwenkwelle 10 eine formschlüssige Verbindung ein, durch die die Kraft zum Schwenken des Albarranhebels 2 vom Endoskopkopf 1 zum Albarranhebel 2 sicher übertragbar ist. Dadurch wird ein einfacher und kostengünstiger Albarranhebel geschaffen, der vom Zugseil 3 räumlich getrennt ist. Ein Albarranhebel bietet aufgrund seines geometrischen Aufbaus viele Hinterschnitte, an denen sich beim Gebrauch Keime etc. ansetzen können, die auch bei einem intensiven Reinigen und Sterilisieren möglicherweise am Albarranhebel verbleiben. Der Albarranhebel 2 kann aber nach einmaligem Gebrauch entsorgt werden. Somit bildet der erfindungsgemäße Albarranhebel 2 die Möglichkeit, dass ein Übertragen von Keimen etc., mit denen ein Endoskop bei einem Einsatz des Endoskops in Kontakt gelangte, beim nächsten Einsatz auf den nächsten Patienten vermieden wird.

Der Albarranhebel 2 kann an bisherigen Endoskopen angewendet werden, die einen entsprechenden Wellenabschnitt 11 haben.

Der Seilzugkanal ist vom Albarranhebel 2 getrennt. Ferner ist der Seilzugkanal von der Kappe getrennt. Somit ist der Seilzugkanal von den Elementen, die der Kontamination ausgesetzt sind und eine relativ komplexe Geometrie mit Hinterschnitten, Kanten etc. haben, getrennt. Im Endoskopkopf ist der Seilzugkanal abgedichtet, wobei das Zugseil gegenüber der Umgebung vollständig abgedichtet ist. Das Abdichten des Seilzugkanals und des Seilzugs ist wasserdicht. Somit wird vermieden, dass Keime in den Seilzugkanal eindringen können oder mit dem Zugseil in Kontakt gelangen können.

### Ausführungsbeispiel 2

Im ersten Ausführungsbeispiel bildet der Einrasthebel 22 das Rastmittel der vorliegenden Erfindung. Im vorliegenden zweiten Ausführungsbeispiel (in den Zeichnungen nicht gezeigt) bildet ein Schieber das Rastmittel der vorliegenden Erfindung.

Die Einpassform 21 ist so aufgebaut, dass sie bei auf dem Wellenabschnitt 11 aufgeschobenem Albarranhebel 2 die Umfangsfläche des Wellenabschnittes 11 von drei Seiten umgibt, d.h. von der proximalen Seite, von oben und von der distalen Seite.

Anstelle des Einrasthebels 22 ist der Schieber an der zur Werkzeugleitfläche 20 entgegengesetzten Seite des Albarranhebels vorgesehen. Der Schieber kann in die proximale Richtung geschoben werden, um die untere Seite der Einpassform 21 zu schließen. Der Schieber kann in die distale Richtung geschoben werden, um die untere Seite der Einpassform 21 zu öffnen.

Auch der Albarranhebel des zweiten Ausführungsbeispiels kann nach dem Einsatz entsorgt werden und liefert die gleichen Effekte und Vorteile wie beim ersten Ausführungsbeispiel.

### Alternativen

Die in Ausführungsbeispiel 1 erwähnte Kappe kann weggelassen werden.

In einer Alternative kann der Einrasthebel 22 des ersten Ausführungsbeispiels anstelle der Rastnase 24 die Form eines Zylinderstiftes haben, die in eine Durchgangsbohrung oder Sacklockbohrung im Wellenabschnitt 11 eingeführt werden kann. In dieser Alternative wird die formschlüssige Verbindung zwischen dem Wellenabschnitt 11 des Kraftübertragungsabschnittes 10 des Endoskopkopfes und der Rastvorrichtung des Albarranhebels 2 durch den Zylinderstift an der proximalen Seite des Einrasthebel 22 und die an die Form des Zylinderstifts angepasste Bohrung im Wellenabschnitt 11 gebildet. In dieser Alternative kann Wellenabschnitt 11 selbst sogar eine Kreisform im Querschnitt (Zylinderform) haben.

Die Erfindung ist bei einem Duodenoskop anwendbar. Das Prinzip der Erfindung kann auch bei einem Ultraschallendoskop und bei jeder anderen Art an Endoskop angewendet werden.

In den Ausführungsbeispielen ist ein Arbeitskanal mit einem Albaranhebel am Ende des Arbeitskanals gezeigt. Die Erfindung kann auch bei Endoskopen angewendet werden, die mehrere Arbeitskanäle mit je einem Albaranhebel am Ende des jeweiligen Arbeitskanals aufweisen.

Die erläuterten Alternativen können kombiniert werden und können bei allen Ausführungsbeispielen angewendet werden.

### Bezugszeichenliste

- 1: Endoskopkopf
- 1A: Optikerstreckung
- 1B: Albarranhebelabstützerstreckung
- 2: Albarranhebel
- 3: Zugseil
- 9: Hebelelement
- 10: Kraftübertragungsabschnitt des Endoskopkopfes
- 11: Wellenabschnitt
- 13: Zugseilkanal
- 17: Kamera
- 18: Beleuchtungseinrichtung
- 20: Werkzeugleitfläche
- 21: Einpassform
- 22: Einrasthebel
- 23: Drehwelle
- 24: Rastnase
- 25: Betätigungseinrichtung

## Patentansprüche

1. Endoskop mit einem Endoskopkopf (1) und einem am Endoskopkopf (1) anbringbaren Albarranhebel (2), wobei der Albarranhebel (2) folgendes aufweist:
eine Werkzeugleitfläche (20), an der ein durch einen Arbeitskanal (12) eines Endoskops führbares Werkzeug in Kontakt treten kann, und
eine Rastvorrichtung (21, 22) zum formschlüssigen Einrasten an einem von einer proximalen Seite des Endoskops betätigbaren Kraftübertragungsabschnitt (10) des Endoskopkopfes (1) des Endoskops, wobei der Kraftübertragungsabschnitt (10) eine Kraft zum Schwenken des Albarranhebels (2) auf den Albarranhebel (2) überträgt, und ein Rastmittel (22) der Rastvorrichtung (21, 22) ein Entfernen des Albarranhebels (2) vom Kraftübertragungsabschnitt (10) des Endoskopkopfes (1) verhindert,
wobei das Rastmittel (22) als am Albarranhebel (2) vorgesehener zum Albarranhebel (2) relativ bewegbarer Einrasthebel (22) ausgebildet ist. **dadurch gekennzeichnet, dass**
der Albarranhebel (2) an der der zur Werkzeugleitfläche (20) entgegengesetzten Seite des Albarranhebels (2) eine Drehwelle (23) hat, wobei der Einrasthebel (22) an der Drehwelle (23) relativ zum Albarranhebel (2) drehbar ist.

2. Endoskop mit einem Endoskopkopf (1) und einem am Endoskopkopf (1) anbringbaren Albarranhebel (2), wobei der Albarranhebel (2) folgendes aufweist:
eine Werkzeugleitfläche (20), an der ein durch einen Arbeitskanal eines Endoskops führbares Werkzeug in Kontakt treten kann, und
eine Rastvorrichtung (21, 22) zum formschlüssigen Einrasten an einem von einer proximalen Seite des Endoskops betätigbaren Kraftübertragungsabschnitt (10) des Endoskopkopfes (1) des Endoskops, wobei der Kraftübertragungsabschnitt (10) eine Kraft zum Schwenken des Albarranhebels (2) auf den Albarranhebel (2) überträgt, und ein Rastmittel (22) der Rastvorrichtung (21, 22) ein Entfernen des Albarranhebels (2) vom Kraftübertragungsabschnitt (10) des Endoskopkopfes (1) verhindert,
**dadurch gekennzeichnet, dass**
das Rastmittel als am Albarranhebel (2) vorgesehener zum Albarranhebel (2) verschiebbarer Schieber ausgebildet ist.

3. Endoskop gemäß Anspruch 1 oder 2, wobei
die Rastvorrichtung (21, 22) an der proximalen Seite des Albarranhebels (2) eine geometrische Form (21) aufweist, die einer Gegenform eines Wellenabschnittes (11) des Kraftübertragungsabschnittes (10) des Endoskopkopfes (1) entspricht,
auf diesen Wellenabschnitt (11) der Albarranhebel (2) aufsetzbar ist, und
das Rastmittel (22) der Rastvorrichtung (21, 22) ein Entfernen des Albarranhebels (2) vom Wellenabschnitt (11) des Kraftübertragungsabschnittes (10) des Endoskopkopfes (1) verhindert.

4. Endoskop gemäß Anspruch einem der Ansprüche 1 bis 3, wobei
das Rastmittel (22) an der zur Werkzeugleitfläche (20) entgegengesetzten Seite des Albarranhebels (2) vorgesehen ist.

5. Endoskop gemäß einem der Ansprüche 1 bis 4, wobei
das Rastmittel (22) an der zur Werkzeugleitfläche (20) entgegengesetzten Seite des Albarranhebels (2) eine Betätigungseinrichtung (25) zur Betätigung des Rastmittels (22) hat.

6. Endoskop gemäß einem der Ansprüche 1 bis 5, wobei
der Albarranhebel (2) aus Kunststoff hergestellt ist.

7. Endoskop gemäß Anspruch 3, wobei
ein Wellenabschnitt (11) des Kraftübertragungsabschnittes (10) des Endoskopkopfes und die Rastvorrichtung (21, 22) des Albarranhebels (2) eine formschlüssige Verbindung eingehen, durch die die Kraft zum Schwenken des Albarranhebels (2) vom Endoskopkopf (1) zum Albarranhebel (2) übertragbar ist.

8. Endoskop gemäß Anspruch 7, wobei
die geometrische Form der Rastvorrichtung (21, 22) des Albarranhebels (2) eine Gegenform zur geometrischen Form des Wellenabschnittes (11) des Kraftübertragungsabschnittes (10) des Endoskopkopfes (1) bildet.

9. Endoskop gemäß einem der Ansprüche 1 bis 8, wobei
das Endoskop eine am Aussenumfang des Endoskopkopfes (1) von der distalen Seite aufsetzbare Kappe hat,
wobei die Kappe von einem Zugseil (3) zur Betätigung des Albarranhebels (2) separat ist.

10. Endoskop gemäß einem der Ansprüche 1 bis 9, wobei
das Endoskop ein Duodenoskop ist.

## Claims

1. An endoscope having an endoscope head (1), and an Albarranlever (2) arrangeable at the endoscope head (1), the Albarranlever (2) comprising
a tool guiding surface (20), wherein at the tool guiding surface (20) a tool guided through a working channel (12) of an endoscope, can be contacted, and
a latching device (21, 22) for form-fitting latching at a force transmitting portion (10) of the endoscope head (1) of the endoscope, said force transmitting portion (10) being actuable from the proximal side of the endoscope, and said force transmitting portion (10) transmitting a force to the Albarranlever (2) for tilting (rotating) the Albarranlever (2), and wherein a latching means (22) of the latching device (21, 22) prevents removing the Albarranlever (2) from the force transmitting portion (10) of the endoscope head (1),
wherein the latching means (22) is formed as a latching lever (22) which is provided at the Albarranlever (2) and is moveable relatively to the Albarranlever (2),
**characterized in that**
the Albarranlever (2) comprises a rotation shaft (23) at the side of the Albarranlever (2) which is opposite to the tool guiding surface (20), wherein the latching lever is pivotable at the rotation shaft (23) relative to the Albarranlever (2).

2. An endoscope having an endoscope head (1), and an Albarranlever (2) arrangeable at the endoscope head (1), the Albarranlever (2) comprising
a tool guiding surface (20), wherein at the tool guiding surface (20) a tool guided through a working channel of an endoscope can be contacted, and
a latching device (21, 22) for form-fitting latching (snapping) at a force transmitting portion (10) of the endoscope head (1) of the endoscope, said force transmitting portion (10) being actuable from the proximal side of the endoscope, and said force transmitting portion (10) transmitting a force to the Albarranlever (2) for tilting (rotating) the Albarranlever (2), and wherein a latching means (22) of the latching device (21, 22) prevents removing the Albarranlever (2) from the force transmitting portion (10) of the endoscope head (1),
**characterized in that**
the latching means (22) is formed as a slider which is provided at the Albarranlever (2) and is slidably relatively to the Albarranlever (2).

3. The endoscope according to claim 1 or 2, wherein
at the proximal side of the Albarranlever (2) the latching device (21, 22) comprises a geometrical shape (21) which corresponds to a counter shape of a shaft portion (11) of the force transmitting portion (10) of the endoscope head (1),
the Albarranlever (2) is fittable to this shaft portion (11), and
the latching means (22) of the latching device (21, 22) prevents removing the Albarranlever (2) from the shaft portion (11) of the force transmitting portion (10) of the endoscope head (1).

4. The endoscope according to one of the claims 1 to 3, wherein
the latching means (22) is provided at the side of the Albarranlever (2) which is opposite to the tool guiding surface (20).

5. The endoscope according to one of the claims 1 to 4, wherein
the latching means (22) comprises an actuation means (25) for actuating the latching means (22), the actuation means (25) being provided at the side of the Albarranlever (2) which is opposite to the tool guiding surface (20).

6. The endoscope according to one of the claims 1 to 5, wherein
the Albarranlever (2) is made of resin.

7. The endoscope according to claim 3, wherein
a shaft portion (11) of the force transmitting portion (10) of the endoscope head (1) and the latching device (21, 22) of the Albarranlever (2) commit a form-fit connection by which the force for pivoting the Albarranlever (2) is transmittable from the endoscope head (1) to the Albarranlever (2).

8. The endoscope according to claim 7, wherein
the geometrical shape of the latching device (21, 22) of the Albarranlever (2) forms a counter shape to the geometrical shape of the shaft portion (11) of the force transmitting portion (10) of the endoscope head (1).

9. The endoscope according to one of the claims 1 to 8, wherein
the endoscope comprises a cap to be set at the outer circumference of the endoscope head (1) from the distal side,
wherein the cap is separate from a control wire (3) for actuating the Albarranlever (2).

10. The endoscope according to one of the claims 1 to 9, wherein
the endoscope is a duodenoscope.

## Revendications

1. Endoscope avec une tête d'endoscope (1) et un levier d'Albarran (2) pouvant être monté sur la tête d'endoscope (1), dans lequel le levier d'Albarran (2) présente ce qui suit :
une surface de guidage d'outil (20), sur laquelle un outil pouvant être guidé à travers un canal de travail (12) d'un endoscope peut entrer en contact, et
un dispositif d'encliquetage (21, 22) pour l'encliquetage par coopération de formes sur une partie de transmission de force (10) de la tête d'endoscope (1) de l'endoscope pouvant être actionnée à partir d'une face proximale de l'endoscope, dans lequel la partie de transmission de force (10) transmet une force au levier d'Albarran (2) pour faire pivoter le levier d'Albarran (2), et un moyen d'encliquetage (22) du dispositif d'encliquetage (21, 22) empêche un éloignement du levier d'Albarran (2) de la partie de transmission de force (10) de l'endoscope (1),
dans lequel le moyen d'encliquetage (22) est réalisé sous la forme d'un levier d'encliquetage (22) prévu sur le levier d'Albarran (2), mobile par rapport au levier d'Albarran (2), **caractérisé en ce que**
le levier d'Albarran (2) possède sur la face du levier d'Albarran (2) opposée à la surface de guidage d'outil (20) un arbre rotatif (23), dans lequel le levier d'encliquetage (22) peut être amené en rotation par rapport au levier d'Albarran (2) sur l'arbre rotatif (23).

2. Endoscope avec une tête d'endoscope (1) et un levier d'Albarran (2) pouvant être monté sur la tête d'endoscope (1), dans lequel le levier d'Albarran (2) présente ce qui suit :
une surface de guidage d'outil (20), sur laquelle un outil pouvant être guidé à travers un canal de travail d'un endoscope peut entrer en contact, et
un dispositif d'encliquetage (21, 22) pour l'encliquetage par coopération de formes sur une partie de transmission de force (10) de la tête d'endoscope (1) de l'endoscope pouvant être actionnée à partir d'une face proximale de l'endoscope, dans lequel la partie de transmission de force (10) transmet une force au levier d'Albarran (2) pour faire pivoter le levier d'Albarran (2), et un moyen d'encliquetage (22) du dispositif d'encliquetage (21, 22) empêche un éloignement du levier d'Albarran (2) de la partie de transmission de force (10) de la tête d'endoscope (1),
**caractérisé en ce que**
le moyen d'encliquetage est réalisé sous la forme d'un coulisseau prévu sur le levier d'Albarran (2), pouvant coulisser par rapport au levier d'Albarran (2).

3. Endoscope selon la revendication 1 ou 2, dans lequel le dispositif d'encliquetage (21, 22) présente sur la face proximale du levier d'Albarran (2) une forme géométrique (21), qui correspond à une forme complémentaire d'une partie d'arbre (11) de la partie de transmission de force (10) de la tête d'endoscope (1),
le levier d'Albarran (2) peut être posé sur cette partie d'arbre (11), et
le moyen d'encliquetage (22) du dispositif d'encliquetage (21, 22) empêche un éloignement du levier d'Albarran (2) de la partie d'arbre (11) de la partie de transmission de force (10) de la tête d'endoscope (1).

4. Endoscope selon l'une quelconque des revendications 1 à 3, dans lequel
le moyen d'encliquetage (22) est prévu sur la face du levier d'Albarran (2) opposée à la surface de guidage d'outil (20).

5. Endoscope selon l'une quelconque des revendications 1 à 4, dans lequel
le moyen d'encliquetage (22) possède sur la face du levier d'Albarran (2) opposée à la surface de guidage d'outil (20) un système d'actionnement (25) pour l'actionnement du moyen d'encliquetage (22).

6. Endoscope selon l'une quelconque des revendications 1 à 5, dans lequel
le levier d'Albarran (2) est fabriqué à partir de matière plastique.

7. Endoscope selon la revendication 3, dans lequel
une partie d'arbre (11) de la partie de transmission de force (10) de la tête d'endoscope et le dispositif d'encliquetage (21, 22) du levier d'Albarran (2) établissent une liaison par coopération de formes, par laquelle la force pour le pivotement du levier d'Albarran (2) peut être transmise de la tête d'endoscope (1) au levier d'Albarran (2).

8. Endoscope selon la revendication 7, dans lequel
la forme géométrique du dispositif d'encliquetage (21, 22) du levier d'Albarran (2) forme une forme complémentaire de la forme géométrique de la partie d'arbre (11) de la partie de transmission de force (10) de la tête d'endoscope (1).

9. Endoscope selon l'une quelconque des revendications 1 à 8, dans lequel
l'endoscope possède un capuchon pouvant être posé sur la périphérique extérieure de la tête d'endoscope (1) à partir de la face distale,
dans lequel le capuchon est séparé d'un câble de traction (3) pour l'actionnement du levier d'Albarran (2).

10. Endoscope selon l'une quelconque des revendications 1 à 9, dans lequel
l'endoscope est un duodénoscope.
